# EUROPEAN PATENT APPLICATION

(11) **EP 3 821 920 A1**
(43) Date of publication of application: **19.05.2021**
(21) Application number: 18926131.6
(22) Date of filing: 13.07.2018
(51) Int. Cl.: A61L 31/04

(54) **COMBINATION FOR ENDOSCOPIC SURGERY**

(71) Applicant: T-ACE Medical Co., Ltd., Tainan City, Taiwan 704 (TW)
(72) Inventor: CHEN, Chih Hong, Tainan, Taiwan 704 (TW); KANG, Jui Wen, Tainan, Taiwan 704 (TW); TSENG, Chung Po, Tainan, Taiwan 704 (TW); KANG, Wun Syuan, Tainan, Taiwan 704 (TW); LIN, Chuan Sheng, Tainan, Taiwan 704 (TW); HO, Chi Ming, Tainan, Taiwan 704 (TW); WEN, Chih Ning, Tainan, Taiwan 704 (TW); LIN, Xi Zhang, Tainan, Taiwan 704 (CN)
(74) Representative: Viering, Jentschura & Partner mbB Patent- und Rechtsanwälte
(86) International application number: PCT/CN2018/095644
(87) International publication number: WO 2020/010616

(57) **Abstract**

A combination or a composition for an endoscopic surgery is a lyophilized powder used in endoscopic mucosal resection or endoscopic submucosal dissection, and comprises a first composition and a second composition. The first composition comprises sodium alginate and a member selected from a group consisting of sucrose and sorbitol. The second composition comprises calcium lactate. The first composition and the second composition are stored in separate containers.

## Description

### BACKGROUND OF THE INVENTION

### Technical Field

The present invention relates to a combination or a composition for endoscopic surgery, in particular to a lyophilized powder used in endoscopic mucosal resection (EMR) or endoscopic submucosal dissection (ESD).

### Related Art

For minimally invasive treatment of gastrointestinal adenomas and early cancers involving minimal risk of lymph node metastasis, endoscopic mucosal resection (EMR) and endoscopic submucosal dissection (ESD) provide new options. The use of submucosal injection solutions is indispensable for the success of these endoscopic resection or dissection techniques (Drug Design, Development and Therapy 2008: 2 131-138).

In the past, normal saline (NS) solutions are often used as submucosal injection solutions. Other submucosal injection solutions have also been developed, such as hypertonic saline solution, glycerin, dextrose water (DW), hyaluronic acid (HA), fibrinogen mixture (FM) and hydroxypropyl methylcellulose (HPMC) (Drug Design, Development and Therapy 2008: 2131-138). These injection solutions have high viscosity when injected into target tissues at high concentrations, thereby forming a cushion during EMR and ESD operations and reducing the outflow of injections from lesion areas due to viscosity.

However, the injection solutions with high viscosity cause difficulties in injection processes; for example, it is difficult to force a viscous solution to flow through an injection device. In addition, currently available hydrogels are unstable during storage, easily decomposed in the presence of water, and not as stable as desired when used *in vivo.* These shortcomings are not conducive to the use of hydrogels as drug delivery carriers or in other biomedical applications.

Therefore, there is a need to provide a novel composition or combination for endoscopic surgery that is easy to obtain, long in shelf life, easy to inject and capable of providing high and durable submucosal cushions.

### SUMMARY

The present invention provides a combination for endoscopic surgery that can be used in tissue lifting or tissue elevation, especially mucosal elevation.

Preferably, the combination of the present invention is an instant powder, which can be used as an injection solution after reconstitution.

Preferably, when the combination of the present invention is an injection solution, the combination can be used as a solution for mucosal elevation in endoscopic surgery.

In one aspect of the present invention, a combination is provided and includes a first composition including an anionic polysaccharide and a solubilizer and a second composition including a cationic crosslinking agent, where the first composition and the second composition are stored in separate containers. Preferably, the first composition and the second composition are in the form of lyophilized powder and stored in separate containers.

In a preferred embodiment of the present invention, the anionic polysaccharide is sodium alginate.

In a preferred embodiment of the present invention, the solubilizer is selected from a group consisting of sucrose and sorbitol.

In another preferred embodiment of the present invention, when the total content of the first composition in powder form is 100 parts by weight, the content of sodium alginate is about 5 to 15 parts by weight, specifically 5, 6, 7, 8, 9, 10, 11, 15, 13, 14, or 15 parts by weight.

In another preferred embodiment of the present invention, when the total content of the first composition in powder form is 100 parts by weight, the content of sucrose is about 25 to 35 parts by weight, specifically 25, 26, 27, 28, 29, 30, 31, 32, 33, 34 or 35 parts by weight.

In another preferred embodiment of the present invention, when the total content of the first composition in powder form is 100 parts by weight, the content of sorbitol is about 60 to 65 parts by weight, specifically 60, 61, 62, 63, 64 or 65 parts by weight.

In a further preferred embodiment of the present invention, when the total content of the first composition in powder form is 100 parts by weight, the content of sodium alginate is about 12.5 parts by weight, the content of sucrose is about 25 parts by weight, and the content of sorbitol is about 62.5 parts by weight.

In a preferred embodiment of the present invention, the cationic crosslinking agent is selected from a group consisting of calcium lactate and mannitol.

In another preferred embodiment of the present invention, when the total content of the second composition in powder form is 100 parts by weight, the content of calcium lactate is about 50 to 70 parts by weight, specifically 50, 51, 52, 53, 54, 55, 56, 57, 58, 59, 60, 61, 62, 63, 64, 65, 66, 67, 68, 69 or 70 parts by weight.

In another preferred embodiment of the present invention, when the total content of the second composition in powder form is 100 parts by weight, the content of mannitol is about 30 to 50 parts by weight, specifically 30, 31, 32, 33, 34, 35, 36, 37, 38, 39, 40, 41, 42, 43, 44, 45, 46, 47, 48, 49 or 50 parts by weight.

In another preferred embodiment of the present invention, when the total content of the second composition in powder form is 100 parts by weight, the content of calcium lactate is about 67 parts by weight, and the content of mannitol is about 33 parts by weight.

In another aspect of the present invention, a combination is provided and includes a first composition including an anionic polysaccharide and a solubilizer and a second composition including a cationic crosslinking agent, where the first composition and the second composition are in the form of solutions and stored in separate containers.

In a preferred embodiment of the present invention, when the total content of the first composition in solution form is 100 parts by weight, the content of sodium alginate is about 0.4 to 1.2 parts by weight, the content of sucrose is about 2.0 to 2.8 parts by weight, and the content of sorbitol is about 3.5 to 5.2 parts by weight; and when the total content of the second composition in solution form is 100 parts by weight, the content of calcium lactate is about 3.0 to 4.2 parts by weight, and the content of mannitol is about 1.8 to 3.0 parts by weight.

In a preferred embodiment of the present invention, when the total content of the first composition in solution form is 100 parts by weight, the content of sodium alginate is about 1 part by weight, the content of sucrose is about 2 parts by weight, and the content of sorbitol is about 5 parts by weight; and when the total content of the second composition in solution form is 100 parts by weight, the content of calcium lactate is about 4 parts by weight, and the content of mannitol is about 2 parts by weight.

In another aspect of the present invention, a method for preparing an injection solution for endoscopic surgery is provided and includes: providing the first composition as described herein; providing the second composition as described herein; and mixing the first composition and the second composition to obtain the injection solution.

In another aspect of the present invention, a method for performing an endoscopic surgery is provided and includes: separately injecting the first composition and the second composition in solution forms into a target tissue of a patient, where the first composition and the second composition are crosslinked in the target tissue of the patient to, preferably, form a hydrogel as a cushion.

In another aspect of the present invention, a combination is provided and includes a first composition and a second composition, where the first composition includes:
(Ia) sodium alginate; and
(Ib) a member selected from a group consisting of sucrose and sorbitol; and
the second composition includes:
(IIa) calcium lactate;
where the first composition and the second composition are stored in separate containers.

In a preferred embodiment of the present invention, the second composition further includes (IIb) mannitol.

In a preferred embodiment of the present invention, when the total content of the first composition in powder form is 100 parts by weight, the content of sodium alginate is about 5 to 15 parts by weight, the content of sucrose is about 25 to 35 parts by weight, and the content of sorbitol is about 60 to 65 parts by weight; and when the total content of the second composition is 100 parts by weight, the content of calcium lactate is about 50 to 70 parts by weight, and the content of mannitol is about 30 to 50 parts by weight.

In a preferred embodiment of the present invention, when the total content of the first composition in powder form is 100 parts by weight, the content of sodium alginate is about 12.5 parts by weight, the content of sucrose is about 25.0 parts by weight, and the content of sorbitol is about 62.5 parts by weight; and when the total content of the second composition is 100 parts by weight, the content of calcium lactate is about 67 parts by weight, and the content of mannitol is about 33 parts by weight.

In a preferred embodiment of the present invention, the first composition and the second composition are in the form of lyophilized powder.

In a preferred embodiment of the present invention, the first composition and the second composition are in the form of solutions.

In a preferred embodiment of the present invention, the reconstitution ratio (solid to water) of the first composition is 0.01: 1, 0.02:1, 0.03:1, 0.04:1, 0.05:1, 0.06:1, 0.07:1, 0.08:1, 0.09:1 and 0.1:1, preferably 0.08:1.

In a preferred embodiment of the present invention, the reconstitution ratio (solid to water) of the second composition is 0.01:1, 0.02:1, 0.03:1, 0.04:1, 0.05:1, 0.06:1, 0.07:1, 0.08:1, 0.09:1 and 0.1:1, preferably 0.06:1.

In a preferred embodiment of the present invention, when the total content of the first composition in solution form is 100 parts by weight, the content of sodium alginate is about 0.4 to 1.2 parts by weight, the content of sucrose is about 2.0 to 2.8 parts by weight, and the content of sorbitol is about 3.5 to 5.2 parts by weight; and when the total content of the second composition in solution form is 100 parts by weight, the content of calcium lactate is about 3.0 to 4.2 parts by weight, and the content of mannitol is about 1.8 to 3.0 parts by weight.

In a preferred embodiment of the present invention, when the total content of the first composition in solution form is 100 parts by weight, the content of sodium alginate is about 1 part by weight, the content of sucrose is about 2 parts by weight, and the content of sorbitol is about 5 parts by weight; and when the total content of the second composition in solution form is 100 parts by weight, the content of calcium lactate is about 4 parts by weight, and the content of mannitol is about 2 parts by weight.

In another aspect of the present invention, application of the combination as described herein in the preparation of a medicament for mucosal elevation in endoscopic surgery is provided.

In a preferred embodiment of the present invention, the medicament is used for endoscopic polypectomy, endoscopic mucosal resection (EMR) or endoscopic submucosal dissection (ESD).

The present invention is described in detail in the following descriptions. Other features, objectives, and advantages of the present invention may be easily found in the detailed descriptions and patent claims of the present invention.

### BRIEF DESCRIPTION OF THE DRAWINGS

FIGs. 1A to 1C show a reconstitution process of a first composition in powder form of the present invention.
FIGs. 2A to 2C show a reconstitution process of a second composition in powder form of the present invention.
FIGs. 3A to 3C show a hydrogel generated by mixing the first composition and the second composition of the present invention.
FIGs. 4A to 4D show application of the combination of the present invention in endoscopic submucosal dissection (ESD).

### DETAILED DESCRIPTION

Unless otherwise defined in this specification, scientific and technical terms used in the present invention should have meanings generally understood by a person skilled in the art. The meaning and scope of the terms should be clear. However, if there is any potential ambiguity, the definition provided in this specification is defined in priority to any dictionary or external definition.

Unless otherwise indicated, the following terms should be understood as having the following meanings according to the usage in the present invention.

Unless otherwise required in the context, singular terms should include plural meanings, and plural terms should include singular meanings. For example, the term "one" is defined as one or more than one.

The term "or" in the claims, unless clearly indicated that the term merely indicates substitution or indicates that substitutions are mutually exclusive, is used for representing "and/or".

Generally, a range in this specification is described as "about" one specific value and/or "about" another specific value. When such range is expressed, an embodiment includes a range from one specific value to another specific value. Similarly, when a value is represented as an approximate value by using a word "about," it should be understood that the specific value from another embodiment. It should be further understood that, endpoint of each range is critical and independent from the other endpoint of the range. The term "about" may refer to ±30%, preferably ±20%, more preferably ±10%, and even more preferably ±5%.

Terms "first" and "second" represent different units (for example, a first composition and a second composition). Terms used herein do not necessarily mean that one unit is prior to another unit in sequence, and merely provide a mechanism for distinguishing specific units.

The term "composition" refers to a mixture of one or more compounds, elements or molecules. The composition may be in the form of solution, tablet, powder, lyophilized powder, pellet, bead, granules microsphere, capsule, pill and the like.

The term "combination" refers to a combination of one or more compositions that may be administered simultaneously, separately or sequentially. In the case of separate or sequential administration, delayed administration of the second composition would not lead to loss of benefits of a combined effect.

The term "lyophilization" refers to a drying process that involves freezing an aqueous material to below 0°C, and reducing the ambient pressure by a vacuum pump to sublime the frozen water in the material.

The term "reconstitution" refers to the conversion of a pharmaceutical composition in solid form into liquid state by addition of a solvent.

The term "solvent" is a substance that dissolves a solute to produce a solution. Suitable solvents include, but are not limited to, saline or water for injection (WFI). The solvent may include a suitable buffer solution (e.g., phosphate, histidine, acetate, citrate and succinate).

The term "pharmaceutically acceptable carrier" refers to a solvent, a diluent, an adhesive, a binder, an adjuvant, an excipient, a receptor, a stabilizer, an analog, a flavoring agent, a sweetener, an emulsifier and/or a preservative that are known in the art and used in the preparation of pharmaceutical compositions. Examples of the pharmaceutically acceptable carrier include, but are not limited to, water, saline, buffer, inert solid and nontoxic solid.

### Solubilizer

The solubilizer may be glycerin, propylene glycol, sorbitol, sucrose, glucose polyethylene glycol, α-hydroxy acid, cyclodextrin (e.g., β-cyclodextrin and γ-cyclodextrin), cyclodextrin derivatives (e.g., sulfobutyl or hydroxypropyl ether), bile acid, bile acid derivatives, sterol derivatives and alcohols. The solubilizer according to the present invention is preferably selected from a group consisting of sorbitol and sucrose, and more preferably includes sorbitol and sucrose.

### Cationic crosslinking agent

The cationic crosslinking agent may be calcium sulfate (CaSO₄), calcium carbonate (CaCO₃), calcium lactate or mannitol. The solubilizer according to the present invention preferably includes calcium lactate or mannitol, and more preferably includes calcium lactate and mannitol.

### Antifreezing agent

The antifreezing agent may be sugar, sugar alcohol and mixtures thereof, where the sugar may be selected from lactose, maltose, sucrose, trehalose and combinations thereof, and the sugar alcohol may be selected from mannitol, sorbitol, maltitol, xylitol, lactitol and combinations thereof. When the total content of the combination is 100 parts by weight, the content of the antifreezing agent is 1 to 20 parts by weight. The solubilizer according to the present invention is preferably mannitol.

### Crosslinking

The first composition and the second composition according to the present invention are crosslinked in a target tissue to preferably form a hydrogel. A method for forming a hydrogel from alginate includes ionic crosslinking, covalent crosslinking and thermal gelation (Prog Polym Sci. 2012 January; 37(1): 106-126).

Ionic crosslinking refers to the use of ionic crosslinking agents, such as divalent cations (namely Ca²⁺), which only chelate with guluronates in an alginate chain due to the high coordination number of the guluronate structure for divalent ions.

Covalent crosslinking refers to the use of poly(ethylene glycol) (PEG), poly(aldeguluronic acid) (PAG), poly(acrylamide-co-hydrazide) (PAH) and adipic acid dihydrazide (AAD) to form a PEG gel, a PAG/PAH gel or a PAG/AAD gel.

Thermal gelation refers to the use of a poly(N-isopropylacrylamide) (PNIPAAm) hydrogel, which exhibits adjustable swelling properties in response to temperature changes.

### Container

The composition of the present invention may be packaged in ampoules, vials, bottles, pre-filled syringes or similar containers. The first composition and the second composition of the present invention are stored in separate containers. For example, the composition of the present invention may be packaged in liquid form in a 5 ml or 10 ml pre-filled syringe, or in a 10 ml, 20 ml or 50 ml ampoule or ampule. The composition of the present invention may be administered by an endoscopic injection needle, preferably at room temperature.

In a preferred embodiment of the present invention, the first composition and the second composition are subjected to lyophilization by a lyophilizer to become porous powder that has large contact areas and can be dissolved for injection upon addition of a suitable amount of WFI. In addition, lyophilization can prevent the powder from oxidation, degradation and deterioration, therefore prolonging the shelf life of the compositions. When in use, the first composition and the second composition are respectively mixed with WFI; when fully dissolved, the compositions become a first composition solution and a second composition solution respectively, which can be used in endoscopic surgery. During injection, because the first composition solution and the second composition solution are not in contact or do not crosslink with each other in the injection tube, no gelatinous substance is formed; therefore, the problem of blocking the injection tube in the prior art is avoided. Now that the present invention has been described in general, the present invention can be more easily understood with reference to the following examples, which provide exemplary schemes for preparation of the combination of the present invention and for use of the combination in endoscopic surgeries. These examples are provided for illustrative purposes only, and are not intended to limit the scope of the present invention in any way. While every effort has been made to ensure the accuracy of the parameters used (e.g., quantity, temperature and the like), certain experimental errors and deviations should be allowed.

### Examples

### Example 1: Preparation of the first composition and the second composition

| Composition | Component |
|---|---|
| First composition | About 12.5 wt% of sodium alginate |
| | About 25.0 wt% of sucrose |
| | About 62.5 wt% of sorbitol |
| Second composition | About 67 wt% of calcium lactate |
| | About 33 wt% of mannitol |

A preparation method of the first composition includes steps for solution preparation and lyophilization.

The components in the above table were weighed, and put into a container filled with 10 ml of WFI. The solution was fully stirred, filtered, and sterilized. The sterilized solution was transferred into a liquid storage tank, sub-packaged, partially plugged, lyophilized, covered and packaged. The lyophilization steps were performed as follows:

1) pre-lyophilizing: the solutions were pre-lyophilized at -40°C for 5 h and the resulting powder was fully compressed;

2) primary drying: after pre-lyophilization, the vacuum pump was turned on to create a vacuum reaching 20 Pa, and the temperature was risen to -30°C and kept for 5 h; thereafter, the temperature was risen to -20°C and kept for 5 h; thereafter, the temperature was risen to -10°C and kept for 10 h; thereafter, the temperature was risen to 0°C and kept for 10 h; thereafter, the temperature was risen to 10°C and kept for 10 h; thereafter, the temperature was risen to -20°C and kept for 10 h;

3) secondary drying: the temperature was gradually risen to 25°C and kept for 5 h.

The obtained lyophilized powder of the first composition can be easily reconstituted with a solvent in 1 min, and the obtained lyophilized powder of the second composition can be easily reconstituted with a solvent in 10 sec. The reconstitution ratio of the first composition was 0.08:1 (solid to water), the reconstitution ratio of the second composition was 0.06:1 (solid to water), and the component ratios of the first composition and the second composition after reconstitution were as follows:

| Composition | Component |
|---|---|
| First composition | About 1 wt% of sodium alginate |
| | About 2 wt% of sucrose |
| | About 5 wt% of sorbitol |
| | About 92 wt% of water |
| Second composition | About 4 wt% of calcium lactate |
| | About 2 wt% of mannitol |
| | About 94 wt% of water |

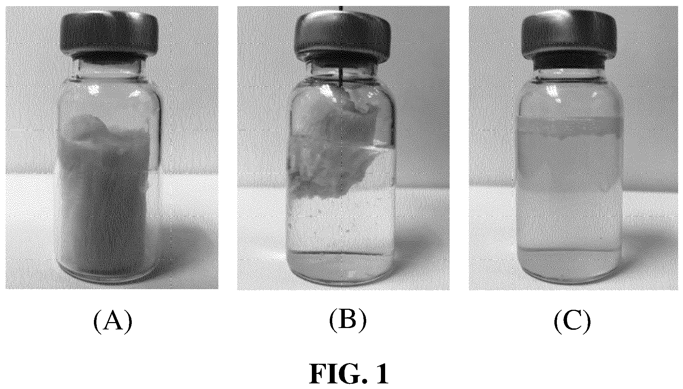

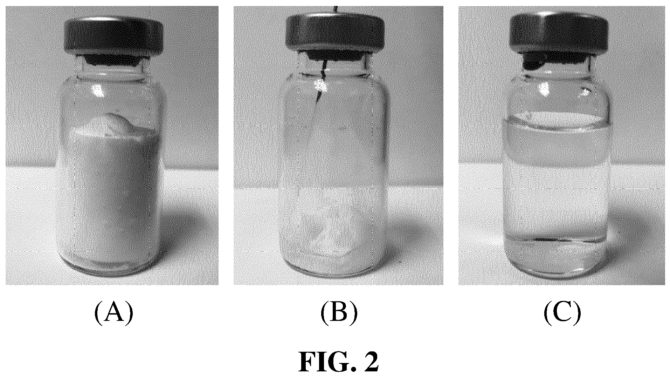

In the examples of dissolution of the instant powders in FIG. 1 and FIG. 2, 10 ml of normal saline was added to the first composition and the second composition separately; after being well shaken, the compositions fully dissolved. FIG. 1A and FIG. 2A show the powder being completely dry, FIG. 1B and FIG. 2B show the powder being in dissolution, and FIG. 1C and FIG. 2C show the powder being completely dissolved. The first composition took about 1 min to change from FIG. 1A to FIG. 1B to FIG. 1C. The second composition took about 5 to 10 sec to change from FIG. 2A to FIG. 2B to FIG. 2C.

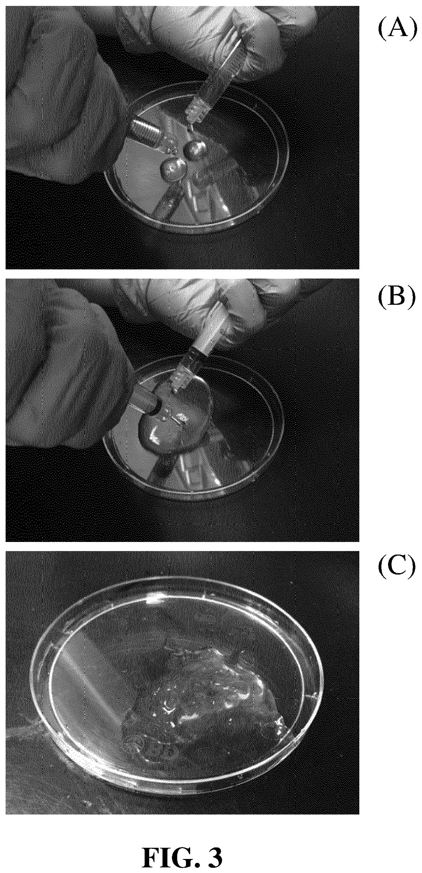

As shown in FIGs. 3A to 3C, when the first composition solution of FIG. 1C and the second composition solution of FIG. 2C were mixed, a hydrogel generated by a crosslinking reaction was observed in 10 sec.

### Example 2: Use of the combination of the present invention in endoscopic surgery

The first composition and the second composition according to Example 1 were prepared as lyophilized powder. During injection, the user administered the first composition or the second composition, and rinsed the endoscopic injection needle to flush out the remaining solution by using about 1 ml of normal saline; the other composition was administered to prevent crosslinking occurred in the catheter.

Alternatively, the first composition and the second composition may be injected simultaneously through two separate needles or by two separate tubes connected to a single needle.

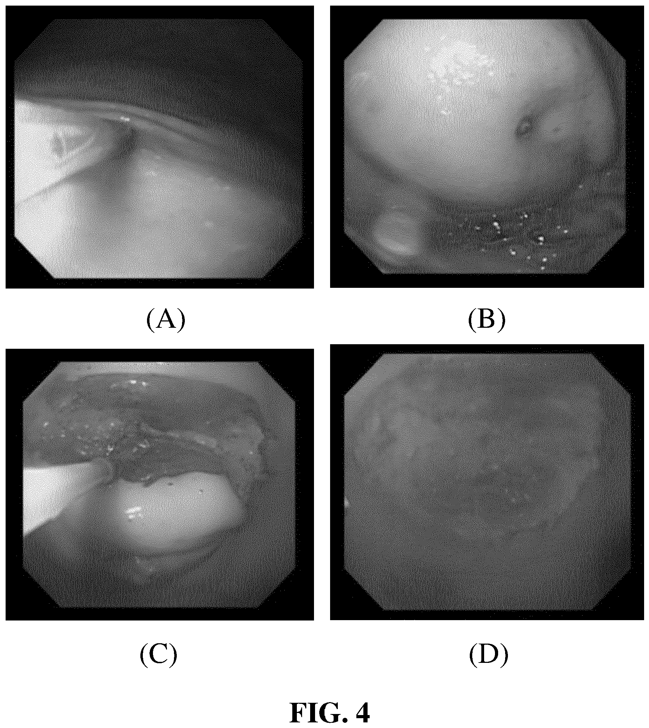

FIGs. 4A to 4D are images showing application of the combination of the present invention in an endoscopic submucosal dissection (ESD) process. FIG. 4A shows a target tissue (injury) before injection, FIG. 4B shows the formation of a cushion from a bulge after injection, FIG. 4C shows endoscopic submucosal dissection of the bulged area, and FIG. 4D shows completion of the endoscopic submucosal dissection surgery. As shown in FIG. 4B, submucosal injection of the combination of the present invention provides high submucosal liquid cushions for safe and effective EMR.

The hydrogel formed by crosslinking of the first composition and the second composition mainly adopts the physical properties of each component in the compositions, rather than utilizing pharmacological, immunological, or metabolic properties to act on human tissues. Therefore, the solutions for endoscopic submucosal dissection of the present invention shall be considered as a medical device.

Additionally, the cushion formed by the solutions for endoscopic submucosal dissection of the present invention is advantageous in its low diffusion in tissues around a lesion.

A person skilled in the art can conceive various modifications and changes of the present invention as those described in the foregoing illustrative embodiments. Therefore, only limitations recorded in the appended claims may be used for limiting the present invention.

## Claims

1. Use of a combination in preparation of a medicament for mucosal elevation in an endoscopic surgery, wherein the combination comprises a first composition and a second composition
the first composition comprises:
(Ia) sodium alginate; and
(Ib) a member selected from a group consisting of sucrose and sorbitol; and
the second composition comprises:
(IIa) calcium lactate;
wherein the first composition and the second composition are stored in separate containers.

2. The use according to claim 1, wherein the second composition further comprises (IIb) mannitol.

3. The use according to claim 1 or 2, wherein:
when the total content of the first composition is 100 parts by weight, the content of sodium alginate is 5 to 15 parts by weight, the content of sucrose is 25 to 35 parts by weight, and the content of sorbitol is 60 to 65 parts by weight; and
when the total content of the second composition is 100 parts by weight, the content of calcium lactate is 50 to 70 parts by weight, and the content of mannitol is 30 to 50 parts by weight.

4. The use according to claim 3, wherein:
when the total content of the first composition is 100 parts by weight, the content of sodium alginate is about 12.5 parts by weight, the content of sucrose is about 25.0 parts by weight, and the content of sorbitol is about 62.5 parts by weight; and
when the total content of the second composition is 100 parts by weight, the content of calcium lactate is about 67 parts by weight, and the content of mannitol is about 33 parts by weight.

5. The use according to claim 1 or 2, wherein the first composition and the second composition are in powder form.

6. The use according to claim 1 or 2, wherein the first composition and the second composition are in solution form.

7. The use according to claim 6, wherein:
when the total content of the first composition in solution form is 100 parts by weight, the content of sodium alginate is 0.4 to 1.2 parts by weight, the content of sucrose is 2.0 to 2.8 parts by weight, and the content of sorbitol is 3.5 to 5.2 parts by weight; and
when the total content of the second composition in solution form is 100 parts by weight, the content of calcium lactate is 3.0 to 4.2 parts by weight, and the content of mannitol is 1.8 to 3.0 parts by weight.

8. The use according to claim 7, wherein:
when the total content of the first composition in solution form is 100 parts by weight, the content of sodium alginate is about 1 part by weight, the content of sucrose is about 2 parts by weight, and the content of sorbitol is about 5 parts by weight; and
when the total content of the second composition in solution form is 100 parts by weight, the content of calcium lactate is about 4 parts by weight, and the content of mannitol is about 2 parts by weight.

9. The use according to claim 1 or 2, wherein the endoscopic surgery is selected from a group consisting of endoscopic polypectomy, endoscopic mucosal resection and endoscopic submucosal dissection.

10. The use according to claim 9, wherein the first composition and the second composition in the medicament are mixed under a mucosa of a tissue to be subjected to the endoscopic surgery.

11. The use according to claim 10, wherein the mixing causes the sodium alginate in the first composition to crosslink with the second composition.
